# FASCICULE DE BREVET EUROPEEN

(11) **EP 0 830 461 B1**
(45) Date de publication et mention de la délivrance du brevet: **09.04.2003**
(21) Numéro de dépôt: 96919863.9
(22) Date de dépôt: 03.05.1996
(51) Int. Cl.: C12S 13/00, A62D 3/00, C12P 1/02, C12P 9/00, C12Q 1/04, C12N 1/16

(54) **PROCEDE DE BIODEGRADATION DES POLYORGANOSILOXANES PAR UN CHAMPIGNON MICROSCOPIQUE ET METHODE DE CRIBLAGE**
VERFAHREN ZUM BIOLOGISCHEN ABBAU VON POLYORGANOSILOXANEN DURCH EINEN MIKROSKOPISCHEN PILZ UND AUFSPÜRUNGSMETHODE
POLYORGANOSILOXANE BIODEGRADATION METHOD USING A MICROSCOPIC FUNGUS AND SCREENING METHOD

(30) Priorité: 05.05.1995 FR 9505613
(43) Date de publication de la demande: 25.03.1998
(73) Titulaire: RHODIA CHIMIE, 92408 Courbevoie Cédex (FR)
(72) Inventeur: BAUD-GRASSET, Frédéric, F-69008 Lyon (FR); PALLA, Jean-Claude, 69003 Lyon (FR)
(74) Mandataire: Bernasconi, Jean
(86) Numéro de dépôt international: FR9600676
(87) Numéro de publication internationale: WO96034986

(56) Documents cités:
- EP-A- 0 192 237
- WO-A-94/21394
- WO-A-94/21854
- WO-A-94/25190
- US-A- 5 085 998
- CHEMICAL ABSTRACTS, vol. 101, no. 17, 22 Octobre 1984 Columbus, Ohio, US; abstract no. 147501, MILSTEIN, OLEG ET AL: "Biodegradation of carbon-14 labeled synthetic lignin polymer by Aspergillus species" XP002012011 & MICROBIOS LETT. (1984), 25(99-100), 113-17 CODEN: MILEDM;ISSN: 0307-5494, 1984,

## Description

La présente invention a trait à un procédé de biodégradation des polyorganosiloxanes (POS) et en particulier des polydiméthylsiloxanes (PDMS). Elle concerne également une méthode de criblage permettant de sélectionner des champignons microscopiques utiles dans le cadre du procédé de biodégradation.

Jusqu'à présent, les silicones étaient considérés de manière générale comme n'étant pas biodégradables.

Cet état de fait constitue aujourd'hui un obstacle à l'utilisation des silicones.

Contre toute attente, les inventeurs ont trouvé que, dans des conditions particulières, il était possible de dégrader les PDMS à l'aide de certains champignons microscopiques appartenant notamment aux genres Phanaerochete et Aspergillus et que cet enseignement pouvait être étendu aux POS en général.

On peut citer plus particulièrement P. sordida et P. chrysosporium, ainsi qu'une souche d'Aspergillus nouvellement isolée, dite Aspergillus sydowii BJS 94 déposée le 22 février 1996 à la CNCM (Collection Nationale de Culture de Microorganismes de l'Institut Pasteur) sous la référence I 1679.

La présente invention a donc pour objet un procédé de biodégradation des POS, en particulier des PDMS, dans lequel on met en contact les POS avec au moins un champignon microscopique, de préférence en présence d'au moins un co-substrat, le champignon microscopique étant choisi notamment dans la famille des Corticiacea, de préférence dans le genre Phanaerochaete, ou dans le genre Aspergillus, les champignons préférés étant choisis dans le groupe consistant en P. sordida, P. chrysosporium et A. sydowii BJS 94.

Ces champignons sont librement accessibles auprès des collections indiquées ci-dessus et dans les exemples.

Le co-substrat est de préférence une source de carbone et d'énergie facilement assimilable par le champignon. Il s'agit en particulier d'un hydrate de carbone ou d'un mélange d'hydrates de carbone et tout particulièrement sucre, de préférence glucose ou saccharose.

Selon un premier mode de réalisation, préféré dans le cas des Phanaerochetes, du procédé selon l'invention, on peut travailler en milieu carencé en azote et de préférence, dans ce cas, à pH acide, de préférence compris entre pH 4,5 et pH 6.

Selon un deuxième mode de réalisation de l'invention, on peut travailler en milieu non carencé en azote et de préférence alors à pH proche de la neutralité.

Par pH proche de la neutralité, on entend notamment un pH compris entre 6,5 et 7,5, de préférence compris entre 6,7 et 7,3 et plus préférentiellement de l'ordre de 7.

Le procédé selon l'invention peut être conduit en culture stationnaire mais l'on préfère toutefois le conduire en culture agitée avec de préférence des concentrations en PDMS allant jusqu'à 5000 mg/l, notamment de 100 à 5000 mg/l, de préférence de 250 à 500 mg/l.

L'ensemencement peut s'effectuer à l'aide de spores, de mycélium ou d'un mélange des deux.

La présente invention ayant pour la première fois prouvé que les POS pouvaient être dégradés par un champignon microscopique, elle a également pour objet une méthode de criblage permettant de sélectionner d'autres champignons microscopiques capables de dégrader les POS, en particulier les PDMS, dans les conditions de l'invention.

Cette méthode de criblage consiste à mettre en contact du POS, de préférence du PDMS, avec un champignon. microscopique-candidat, de préférence, en présence d'un co-substrat tel que défini plus haut.

La méthode de criblage peut comprendre en sus un test préalable permettant d'effectuer une présélection de candidats. Ce test préalable consiste à évaluer la capacité des candidats à se développer sur du POS, préférentiellement PDMS, comme unique source de carbone. Dans ce cas, les candidats répondant positivement au test préalable sont ensuite essayés dans la méthode de criblage proprement dite.

Les candidats répondant positivement à la méthode de criblage sont utilisables dans le procédé de biodégradation défini plus haut.

Le procédé de biodégradation défini plus haut peut donc aussi être mis en oeuvre à l'aide des champignons révélés par la méthode de criblage, c'est-à-dire répondant positivement à cette méthode.

Les champignons selon l'invention pourront notamment aussi être utilisés pour la réalisation d'une culture de champignons pour le traitement d'effluents, notamment dans l'industrie des silicones, ou en catalyse enzymatique à des fins de modification de structures chimiques ou de synthèse de dérivés ou de nouveaux produits utiles dans le domaine de l'alimentation, de la pharmacie, etc. Aux fins de la catalyse enaymatique, l'on pourra contrôler les paramètres de la dégradation (quantité de champignon, température, milieu de culture, pH, agitation, etc.)

L'invention va être maintenant décrite plus en détail à l'aide d'exemples de réalisation non limitatifs du procédé selon l'invention et de la figure unique.

Cette figure représente la dégradation du PDMS 100cs par deux souches différentes par comparaison avec un témoin, avec, en abcisse, le temps en jours et, en ordonnée, le taux de biodégradation du PDMS 100cs en %.

### EXEMPLE 1 : criblage de candidats.

On teste (test préalable) une série de champignons microscopiques sur leur capacité à croître en milieu liquide en présence de PDMS comme unique source de carbone. La croissance mycélienne est évaluée au bout de deux semaines. On peut observer une croissance soit à l'interface entre la phrase aqueuse et le PDMS, soit dans le PDMS.

Les champignons sélectionnés font ensuite l'objet de la méthode de criblage selon l'invention. La description suivante d'exemples de mise en oeuvre de procédé de biodégradation est aussi une illustration de la méthode de criblage de l'invention.

### EXEMPLE 2 : Cet exemple est conduit en culture stationnaire :

- incubation dans des flacons bouchés de 100 ml.
   - milieu minéral pour champignon 20 ml (milieu Czapeck modifié).
- glucose comme co-substrat 10 g/l.
- solution de spores (absorbtion à 650 nm = 0,4)
   - huile silicone PDMS 100cs ou 1000cs (Rhodorsil® PDMS 47 V100 et V1000, Rhône Poulenc) : 5 à 10 g/l.
   - alimentation en O₂ pur.

L'analyse des essais est effectuée par deux extractions successives par le pentane (voir exemple 3), suivies par une chromatographie de perméation sur gel permettant de déterminer qualitativement (poids moléculaire) et quantitativement la concentration restante en PDMS par comparaison de la surface des chromatogrammes correspondant aux échantillons avec ceux de témoins.

Aspergillus sydowii BJS94 est facile à cultiver (peut être cultivé sur gélose malt 50 g/l et agar 20 g/l ; ensemencement par spores en suspension ; incubation à 22°C) et sporule abondamment, ce qui permet, lors de la mise en oeuvre du procédé de biodégradation, d'ensemencer avec des spores. Avec ce champignon, on a obtenu les résultats présentés dans le tableau suivant :

| concentration en PDMS | % de biodégradation à 60 jours | déviation standard % |
|---|---|---|
| 10 g/l 100cs | 17 | 10 |
| 5 g/l 100cs | 50 | 11 |
| 5 g/l 1000cs | 37 | 15 |

### EXEMPLE 3 : cet exemple est conduit en culture agitée :

### MATERIEL

- souche P. sordida : disponible auprès de FPL (Forest Products Laboratory) US Department of Agriculture Madison USA Wisconsin 53705 sous la référence (KARST.) ER::KSF. and R.y.v.
- souche P. chrysosporium : disponible auprès de DMS, Grisebachstrasse 8, 3400 Göttingen, Allemagne, sous la référence DSM 1547.
- polydiméthylsiloxane (PDMS) : huile 100 à 1000cs (Rhodorsil®, Rhône Poulenc).

### PRINCIPE :

Dans des erlenmeyers de 500 ml, on introduit 200 ml de milieu minéral pour champignon (milieu carencé en azote selon Ming Tien et T. Kent Kirk, Ed. Wood Willis A. et Kellogg Scott T., Methods in Enzymology : Biomass, part B, lignin, pectin and chitin. San Diego, Ca, Acad. Press Inc., pages 238-249, Vol. 161, 1988), une source de carbone qui est du glucose à 10 g/l, d'une solution de spores de P. sordida ou de P. chrysosporium en suspension (absorbtion à 650 mn = 0,4) et de PDMS (250 ou 500 mg/l).

Les flacons sont placés sur un plateau d'agitation (105 secousses/min), à raison de trois essais par traitement (incubation en milieu carencé en azote et pH entre 4,5 et 6).

### DOSAGES :

Des dosages sont prévus à 0 jour, 7 jours, 15 jours, 30 jours, 60 jours.

### PLAN D'EXPERIENCE :

Extraction au pentane, puis dosage par GPC (chromatographie par perméation sur gel).

### TECHNIQUE D'EXTRACTION :

- ajout de 50 ml de pentane.
- agitation manuelle.
- dans une ampoule à décanter, agitation pendant 20 minutes (sans biomasse).
   - décantation.
   - ajout de 50 ml de pentane dans la biomasse.
   - ultra-sons 5 min à 140 V.
   - dans des ampoules à décanter, agitation pendant 20 minutes.
   - décantation.
   - récupération du pentane à doser.

### RESULTAT :

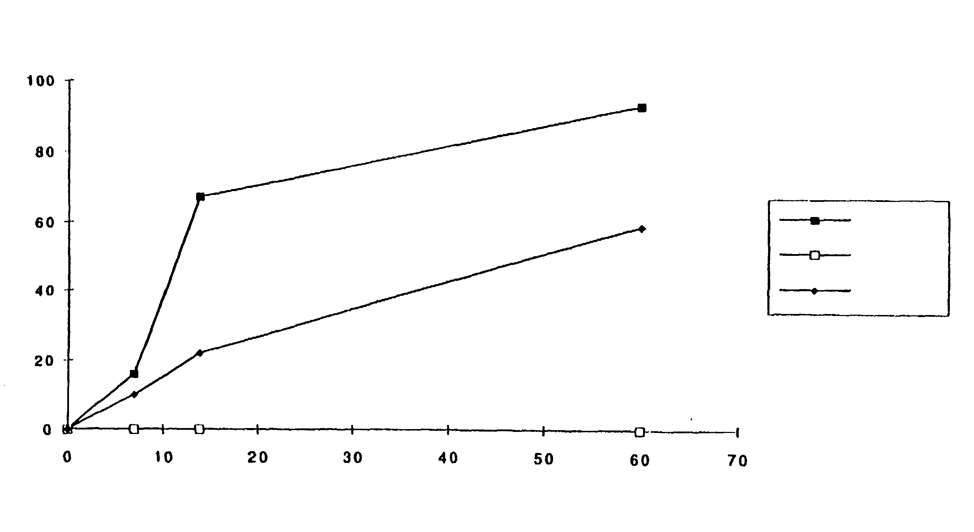

Les résultats du PDMS V100 sont réunis sur la figure 1 par comparaison avec un témoin préparé dans les mêmes conditions mais en l'absence de champignon.
Souche RP3 = P. sordida
Souche RP1 = P. chrysosporium
La figure 1 montre clairement le pouvoir de dégradation important de RP3 et RP1.

### EXEMPLE 4 :

Cet exemple concerne Aspergillus sydowii BJS 94 utilisé en culture agitée sur du PDMS 100cs. On reprend le milieu minéral de l'exemple 2 et l'on incube à pH proche de la neutralité dans des erlenmeyers de 500 ml placés sur un plateau d'agitation, contenant 200 ml du milieu minéral, du glucose à 10 g/l, des spores (absorption à 650 nm = 0,4) et l'huile PDMS 500 mg/l.

| concentration en PDMS | % de biodégradation à 30 jours | déviation standard % |
|---|---|---|
| 500 mg/l PDMS 100 cs | 47 | 12 |

## Revendications

1. Procédé de biodégradation de polyorganosiloxanes (POS), en particulier polydiméthylsiloxanes (PDMS), dans lequel on met en contact les POS avec au moins un champignon microscopique, de préférence en présence d'au moins un co-substrat, le champignon microscopique étant choisi dans la famille des Corticiacea, de préférence dans le genre Phanaecrochaete, ou dans le genre Aspergillus.

2. Procédé selon la revendication 1, **caractérisé en ce que** le champignon est choisi parmi le groupe consistant en Phanaerochaete sordida, Phanerochaete chrysosporium et Apsergillus sydowii BJS94.

3. Procédé selon la revendication 1 ou 2, **caractérisé en ce que** la co-substrat est une source de carbone et d'énergie facilement assimilable par le champignon.

4. Procédé selon la revendication 3, **caractérisé en ce que** le co-substrat est un hydrate de carbone ou un mélange d'hydrates de carbone.

5. Procédé selon la revendication 4, **caractérisé en ce que** le co-substrat est un sucre, de préférence glucose ou saccharose.

6. Procédé selon l'une quelconque des revendications 1 à 5, **caractérisé en ce que** l'on travaille en milieu carencé en azote.

7. Procédé selon la revendication 6, **caractérisé en ce que** l'on travaille à pH acide, de préférence entre pH 4,5 et pH 6.

8. Procédé selon l'une quelconque des revendications 1 à 5, **caractérisé en ce que** l'on travaille en milieu non carencé en azote et à pH proche de la neutralité.

9. Procédé selon l'une quelconque des revendications 1 à 8, **caractérisé en ce que** l'on travaille en culture agitée de POS en concentration allant de 100 à 5000 mg/l, de préférence de 250 à 500 mg/l.

10. Méthode de criblage dans laquelle on met en contact un champignon microscopique avec du POS. de préférence du PDMS, de préférence en présence d'un co-substrat, et l'on évalue la capacité de ce champignon à dégrader le POS.

11. Méthode de criblage selon la revendication 10, **caractérisée en ce que** le co-substrat est une source de carbone et d'énergie facilement assimilable par les champignons microscopiques, de préférence un hydrate de carbone.

12. Méthode de criblage selon la revendication 11, **caractérisée en ce que** le co-substrat est un sucre, de préférence glucose ou saccharose.

13. Procédé de biodégradation selon l'une quelconque des revendications 1 à 9, dans lequel on utilise un champignon répondant positivement à la méthode selon l'une des revendications 10 à 12, à la place, ou en sus, de P. sordida ou P. chrysosporium.

14. Isolat d'Aspergillus sydowii BJS94 déposé à la CNCM sous la référence I-1679.

15. Utilisation des champignons microscopiques Corticiacea et Aspergillus répondant positivement à la méthode selon l'une des revendications 10 à 12 pour le traitement d'effluents en vue de la biodégradation de polyorganosiloxanes, en particulier polydiméthylsiloxanes ou pour la catalyse enzymatique appliquée à la biodégradation de polyorganosiloxanes, en particulier de polydiméthylsiloxanes.

## Patentansprüche

1. Verfahren zum biologischen Abbau von Polyorganosiloxanen (POS), insbesondere Polydimethylsiloxanen (PDMS), wobei die POS mit mindestens einem mikroskopischen Pilz, vorzugsweise in Anwesenheit mindestens eines Co-Substrats, zusammengebracht werden, wobei der mikroskopische Pilz aus der Familie der Corticiaceae, vorzugsweise der Gattung Phanaerochaete oder der Gattung Aspergillus, ausgewählt ist.

2. Verfahren nach Anspruch 1, **dadurch gekennzeichnet, dass** der Pilz aus der Gruppe ausgewählt ist, die aus Phanaerochaete sordida, Phanaerochaete chrysosporium und Aspergillus sydowii BJS94 besteht.

3. Verfahren nach Anspruch 1 oder 2, **dadurch gekennzeichnet, dass** das Co-Substrat eine Quelle für Kohlenstoff und durch den Pilz leicht assimilierbare Energie ist.

4. Verfahren nach Anspruch 3, **dadurch gekennzeichnet, dass** das Co-Substrat ein Kohlenhydrat oder ein Gemisch von Kohlenhydraten ist.

5. Verfahren nach Anspruch 4, **dadurch gekennzeichnet, dass** das Co-Substrat ein Zucker, vorzugsweise Glucose oder Saccharose, ist.

6. Verfahren nach einem der Ansprüche 1 bis 5, **dadurch gekennzeichnet, dass** in einem an Stickstoff verarmten Medium gearbeitet wird.

7. Verfahren nach Anspruch 6, **dadurch gekennzeichnet, dass** bei saurem pH, vorzugsweise von pH 4,5 bis pH 6, gearbeitet wird.

8. Verfahren nach einem der Ansprüche 1 bis 5, **dadurch gekennzeichnet, dass** in einem nicht an Stickstoff verarmten Medium und bei nahezu neutralem pH gearbeitet wird.

9. Verfahren nach einem der Ansprüche 1 bis 8, **dadurch gekennzeichnet, dass** in einer Schüttelkultur von POS in einer Konzentration von 100 bis 5000 mg/l, vorzugsweise 250 bis 500 mg/l, gearbeitet wird.

10. Screeningverfahren, wobei ein mikroskopischer Pilz mit POS, vorzugsweise PDMS, vorzugsweise in Anwesenheit eines Co-Substrats, zusammengebracht wird, und die Fähigkeit des Pilzes zum Abbau von POS untersucht wird.

11. Screeningverfahren nach Anspruch 10, **dadurch gekennzeichnet, dass** das Co-Substrat eine Quelle für Kohlenstoff und durch die mikroskopischen Pilze leicht assimiIierbare Energie ist, vorzugsweise ein Kohlenhydrat.

12. Screeningverfahren nach Anspruch 11, **dadurch gekennzeichnet, dass** das Co-Substrat ein Zucker, vorzugsweise Glucose oder Saccharose, ist.

13. Verfahren zum biologischen Abbau nach einem der Ansprüche 1 bis 9, wobei ein Pilz, der auf das Verfahren nach einem der Ansprüche 10 bis 12 positiv reagiert, anstelle oder neben P. sordida oder P. chrysosporium verwendet wird.

14. Isolat von Aspergillus sydowii BJS94, das bei der CNCM unter der Nummer I-1679 hinterlegt ist.

15. Verwendung der mikroskopischen Pilze Corticiacea und Aspergillus, die auf das Verfahren nach einem der Ansprüche 10 bis 12 positiv reagieren, zur Behandlung von Abwässern zum biologischen Abbau von Polyorganosiloxanen, insbesondere Polydimethylsiloxanen, oder zur enzymatischen Katalyse, die für den biologischen Abbau von Polyorganosiloxanen, insbesondere Polydimethylsiloxanen, angewendet wird.

## Claims

1. Process for the biodegradation of polyorganosiloxanes (POS), particularly polydimethylsiloxanes (PDMS), in which the POS are contacted with at least one microscopic fungus, preferably in the presence of at least one co-substrate, the microscopic fungus being chosen from within the family or Corticiacea, preferably in the genus Phanaerochaete or in the genus Aspergillus.

2. Process according to claim 1, **characterized in that** the fungus is chosen from within the group consisting of Phanaerochaete sordida, Phanaerochaete chrysosporium and Aspergillus sydowii BJS 94.

3. Process according to claim 1 or 2, **characterized in that** the co-substrate is a source of carbon and energy easily digestible by the fungus.

4. Process according to claim 3, **characterized in that** the co-substrate is a carbohydrate or a carbohydrate mixture.

5. Process according to claim 4, **characterized in that** the co-substrate is a sugar, preferably glucose or saccharose.

6. Process according to any one of the claims 1 to 5, **characterized in that** working takes place in a nitrogen-deficient medium.

7. Process according to claim 6, **characterized in that** working takes place with an acid pH, preferably between pH 4.5 and 6.

8. Process according to any one of the claims 1 to 5, **characterized in that** working takes place in a non-nitrogen-deficient medium and with a pH close to neutrality.

9. Process according to any one of the claims 1 to 8, **characterized in that** working takes place in an agitated POS culture with a concentration from 100 to 5000 mg/l, preferably 250 to 500 mg/l.

10. Screening method in which a microscopic fungus is contacted with POS, preferably PDMS, preferably in the presence of a co-substrate, and the capacity of said fungus to degrade POS is evaluated.

11. Screening method according to claim 10, **characterized in that** the co-substrate is a source of carbon and energy easily digestible by the microscopic fungi and preferably a carbohydrate.

12. Screening method according to claim 11, **characterized in that** the co-substrate is a sugar, preferably glucose or saccharose.

13. Biodegradation process according to any one of the claims 1 to 9, in which use is made of a fungus responding positively to the method according to one of the claims 10 to 12, in place of or in addition to P. sordida or P. chrysosporium.

14. Aspergillus sydowii BJS 94 isolate deposited at the CNCM under the reference I-1679.

15. Use of the microscopic fungi Corticiacea and Aspergillus responding positively to the method according to one of the claims 10 to 12 for the treatment of effluents with a view to the biodegradation of polyorganosiloxanes, particularly polydimethylsiloxanes or for enzymatic catalysis applied to the degradation of polyorganosiloxanes, particularly polydimethylsiloxanes.
